# NEUE EUROPÄISCHE PATENTSCHRIFT

(11) **EP 0 190 292 B2**
(45) Veröffentlichungstag und Bekanntmachung des Hinweises auf die Entscheidung über den Einspruch: **13.11.1996**
(45) Hinweis auf die Patenterteilung: 05.06.1991
(21) Anmeldenummer: 85904062.8
(22) Anmeldetag: 23.08.1985
(51) Int. Cl.: A61K 31/44, A61K 9/12

(54) **PHARMAZEUTISCHE ZUBEREITUNG**
PHARMACEUTICAL PREPARATION
PREPARATION PHARMACEUTIQUE

(30) Priorität: 23.08.1984 AT 2708/84
(43) Veröffentlichungstag der Anmeldung: 13.08.1986
(73) Patentinhaber: Kuhlemann & Co., A-1030 Wien (AT)
(72) Erfinder: BURGHART, Walter, A-1030 Wien (AT); BURGHART, Kurt, D-2217 Rosdorf (DE)
(74) Vertreter: Haffner, Thomas M., Dr.
(86) Internationale Anmeldenummer: AT8500027
(87) Internationale Veröffentlichungsnummer: WO8601405

(56) Entgegenhaltungen:
- EP-A- 0 004 650
- EP-A- 0 055 397
- EP-A- 0 117 888
- GB-A- 970 027
- LU-A- 65 929
- Chemical Abstracts, Vol. 97, No. 3, 19 July 1982, Columbus, Ohio (US), p. 58 no. 16919v
- Chemical Abstracts, Vol. 98, No. 17, 25 April 1983, Columbus, Ohio (US), p. 47, no. 137459z
- Chemical Abstracts, Vol. 97, No. 10, 6 September 1982, Columbus, Ohio (US), p. 423, no. 78923a
- Chemical Abstracts, Vol. 98, No. 26, 27 June 1983, Columbus, Ohio (US), p. 383, no. 221832y
- Journal of Pharmaceutical Sciences, Vol. 64, No. 3, March 1975, Washington DC (US) W. Feinstein et al.: "Development and Evaluation of a Dexamethasone Timed-Release Aerosol Formulation"
- Deutsche Apothekerzeitung vol. 128, no. 23, 09.06.88, p.38
- WHO Drug Information vol. 2, no.3, 1988: "Nifedipine:sublingual adsorption ineffective"

## Beschreibung

Die Erfindung bezieht sich auf eine dosierbar versprühbare, pharmazeutische Zubereitung mit einem in einem Lösungsmittel gelösten Wirkstoff. Nifedipin ist als Koronartherapeutikum bekannt und Nifedipin enthaltende feste Präparatzusammensetzungen sowie Verfahren zu ihrer Herstellung sind beispielsweise aus der DE-OS 28 22 882 bekannt geworden. Bei Nifedipin handelt es sich um 4-(2-nitrophenyl)-2,6-dimethyl-3,5-dicarbomethoxy-1,4-dihydropyridin und es ist weiters bereits bekannt, daß diese Verbindung in hohem Maße lichtempfindlich ist. Es sind daher bereits eine Reihe von Vorschlägen gemacht worden, wie die Verabreichung dieser Verbindung erfolgen soll, um die mit der Zersetzung unter Lichteinfluß verbundene Abnahme der Wirksamkeit bzw. den Verlust an Wirksamkeit zu beseitigen. Im besonderen ist aus der DE-OS 28 22 882 eine Präparatzusammensetzung zu entnehmen, bei welcher Nifedipin als feste Präparatzusammensetzung mit Polyvinylpyrrolidonmethylzellulose, Hydroxypropylzellulose und Hydroxypropylmethylzellulose vermengt wird, wobei zusätzlich verschieden oberflächenaktive Mittel, insbesondere Öle, angewandt wurden. Diese Verabreichungsform wurde in erster Linie im Hinblick auf die Verbesserung der Verfügbarkeit des Arzneistoffes nach der Resorption gewählt und ist weiterhin mit erheblichen Nachteilen bezüglich der Stabilität unter Lichteinfluß verbunden.

Aus den Chemical Abstracts Nr. 97:16919v (1982) ist die Behandlung von Histamin induzierten Brochialverengungen mit entweder intravenös oder inhalatorisch verabreichten ethanolischen Nifedipinlösungen bei Hunden bekanntgeworden. In den Chemical Abstracts Nr. 98:137459z (1983) ist ebenfalls eine Inhalation von nifedipinhaltigen Zusammensetzungen bei Hunden beschrieben.

Zur oralen Verabreichung sind neben Tabletten und Pillen auch schon Kapseln vorgeschlagen worden, wobei bei diesen Kapseln Farbstoffe in die Kapselhülle einzuarbeiten sind, um die Lichtzersetzung hintanzuhalten. Eine derartige Zusammensetzung, welche neben dem Wirkstoff noch Polyalkylenglykole und mehrere Alkohole enthält, ist beispielsweise der DE-PS 22 09 526 bzw. der LU-A 65 929 zu entnehmen, wobei als Farbstoff Gelb-Orange S 15985 neben einem Opakisierungsmittel in der Kapselhülle gewählt wurde. Derartige Kapselhüllen sind in der Regel temperaturempfindlich, und es ist auch in diesem Fall eine vollständige Sicherheit gegen Zersetzung der wirksamen Substanz nicht gegeben.

Die Erfindung zielt nun darauf ab, eine pharmazeutische Zubereitung der eingangs genannten Art zu schaffen, bei welcher ein hohes Maß an Sicherheit in bezug auf die Zersetzung der wirksamen Substanz erzielt wird und welche es ermöglicht, die Wirkungsdauer bzw. die Geschwindigkeit des Ansprechens des Medikamentes in weiten Grenzen einzustellen. Die pharmazeutische Zubereitung soll hiebei den Vorteil bieten, daß sie uneingeschränkt und ohne Zuhilfenahme ärztlicher Hilfe rasch verabreicht werden kann. Zur Lösung dieser Aufgabe besteht die Erfindung im wesentlichen darin, daß Nifedipin in einem Lösungsmittel, ausgewählt aus der Gruppe Polyäthylenglykole, insbesondere der Mischungen aus Polyäthylenglykol 400 und 600, partielle Fettsäureester des Sorbitans bzw. des Polyhydroxyäthylensorbitans, sowie Polyvinylpyrrolidone, Polyvinylalkohole, Polyhydroxyäthylenfettalkoholäther bzw. Polyhydroxyäthylenfettsäureester sowie Polyhydroxyäthylen-polyhydroxypropylenkondensate und im besonderen Glycerin-Polyäthylenglykoloxystearat gelöst ist, wobei sie 2 bis 5 Gew.-% Nifedipin, 25 bis 40 Gew.-% Lösungsmittel, 40 bis 25 Gew.-% Äthanol und 30 bis 50 Gew.-% Treibmittel enthält, wobei das Treibmittel gegebenenfalls teilweise durch Diäthyläther ersetzt ist. Dadurch, daß in einem Lösungsmittel, ausgewählt aus der Gruppe Polyäthylenglykole, insbesondere der Mischungen aus Polyäthylenglykol 400 und 600, partielle Fettsäureester des Sorbitans bzw. des Polyhydroxyäthylensorbitans, sowie Polyvinylpyrrolidone, Polyvinylalkohole, Polyhydroxyäthylenfettalkoholäther bzw. Polyhydroxyäthylenfettsäureester sowie Polyhydroxyäthylenpolyhydroxypropylenkondensate und im besonderen Glycerin-Polyäthylenglykoloxystearat 2 bis 5 Gew.-% Wirkstoff gelöst, gegebenenfalls gemeinsam mit pharmazeutischen Hilfsstoffen, insbesondere Aromastoffen. Weichmachern, und weiteren Lösungsmitteln, wie Alkohol, als Aerosol vorliegen, kann der Wirkstoff in einer lichtdichten Verpackung vorliegen, und im Falle eines Versprühens unmittelbar und rasch zur Wirkung gelangen. Bei der Verwendung von Treibmittel, beispielsweise vor halogenierten Kohlenwasserstoffen als Treibmittel, sind jedoch eine Reihe von Randbedingungen zu beachten, um sicherzustellen, daß ein Ausfällen von Nifedipin oder eine Phasentrennung verhindert wird, wobei besonders bevorzugt für eine reproduzierbare Dosierung und die Anwendung von Treibgas 2 bis 5 Gew.% Nifedipin, 25 bis 40 Gew.% Polyäthylenglykol, 40 bis 25 Gew.% Äthanol und 30 bis 50 Gew.%, vorzugsweise etwa 35 Gew.% Treibmittel, bei der dosierbar versprühbaren Zubereitung enthalten sind.

Zur Erzielung einer für inhalatorische Zwecke geeigneten dosierbar versprühbaren, pharmazeutischen Zubereitung wird so vorgegangen, daß Nifedipin in einem Lösungsmittel, ausgewählt aus der Gruppe Polyäthylenglykole, insbesondere der Mischungen aus Polyäthylenglykol 400 und 600, partielle Fettsäureester des Sorbitans bzw. des Polyhydroxyäthylensorbitans, sowie Polyvinylpyrrolidone, Polyvinylalkohole, Polyhydroxyäthylenfettalkoholäther bzw. Polyhydroxyäthylenfettsäureester sowie Polyhydroxyäthylenpolyhydroxypropylenkondensate und im besonderen Glycerin-Polyäthylenglykoloxystearat gelöst ist, wobei sie 0,2 - 5 Gew. Nifedipin, 2 - 20 Gew.-% Lösungsmittel, 0 - 25 Gew.-% Diäthyläther und/oder Halogenkohlenwasserstoff, Rest Äthanoltreibmittelgemische enthält.

Eine geeignete Dosis für verschiedene Formen der Anwendung läßt sich dadurch erzielen, daß die Wirkstoffdosis als gelöster Partikel für inhalatorische Anwendung je Applikation mit 0,2 - 5 mg, vorzugsweise 0,5 - 5 mg, und für sublinguale Anwendung, mit 2-20 mg je Applikation gewählt wird. Die Resorption der Aerosols bei inhalatorischer oder sublingualer Anwendung kann überaus rasch erfolgen, wobei als Trägergasstrom in bekannter Weise komprimierte Luft verwendet werden kann.

Als Trägergasstrom kommen übliche inerte Trägergase bzw. Treibgase, wie z.B. halogenierte Kohlenwasserstoffe in Frage.

In einfacher Weise ist der Wirkstoff in einem pharmazeutisch unbedenklichen Lösungsmittel, insbesondere Polyalkoholen, gelöst und als Lösung im Trägergasstrom suspendiert. Durch Auswahl eines geeigneten unbedenklichen Lösungsmittels aus Polyäthylenglykole, insbesondere der Mischungen aus Polyäthylenglykol 400 und 600, partielle Fettsäureester des Sorbitans bzw. des Polyhydroxyäthylensorbitans, sowie Polyvinylpyrrolidone, Polyvinylalkohole, Polyhydroxyäthylenfettalkoholäther bzw. Polyhydroxyäthylenfettsäureester sowie Polyhydroxyäthylenpolyhydroxypropylenkondensate und im besonderen Glycerin-Polyäthylenglykoloxystearat,läßt sich auch mit relativ einfachen Einrichtungen die gewünschte Dosierung sicherstellen.

Zur Erzielung einer Tröpfchengröße mit Durchmessern zwischen 0,2 und 10µ, bevorzugt 0,5 bis 5µ, kann mit Vorteil so vorgegangen werden, daß die gelösten Teilchen in der Verpackung bereits als Emulsion vorliegen. Eine derartige Emulsion kann hiebei so eingestellt werden, daß die gelösten Partikel entweder bereits in der Emulsion mit Partikelgrößen von 0,2 bis 10µ vorliegen, oder aber unmittelbar nach dem Ausstoß im Trägergasstrom mit Teilchengrößen von 0,2 bis 10µ, vorzugsweise 0,5 bis 5µ vorliegen.

In besonders einfacher Weise läßt sich ein derartiges Präparat dadurch verabreichen, daß die Wirksubstanz mit einem pharmazeutisch unbedenklichen gasförmigen Träger versprüht wird. Für diese Form der Verabreichung können konventionelle Einrichtungen, wie sie beispielswise für die Verabreichung von Nitroglycerin als Aerosol bereits bekannt geworden sind, eingesetzt werden, wobei lediglich eine geeignete Dosierung sichergestellt werden muß.

Die Erfindung wird nachfolgend an Hand von Ausführungsbeispielen näher erläutert.

### Beispiel 1:

Für die Herstellung eines Treibgassprays wurden eine Reihe verschiedener Mischungen erprobt. Bei einer größeren Anzahl derartiger Formulierungen wurde beobachtet, daß Nifedipin auskristallisiert oder eine Phasentrennung bzw. Entmischung auftrat. Eine stabile und reproduzierbar zu dosierende Zubereitung konnte nur innerhalb der nachfolgenden Bereichsgrenzen gefunden werden: 2 bis 5 Gew.% Nifedipin, 25 bis 40 Gew.% Polyäthylenglykol, 40 bis 25 Gew.% Äthanol und 30 bis 50 Gew.%, vorzugsweise etwa 35 Gew.% Treibmittel.

Als Treibgase können im Rahmen der vorliegenden Erfindung neben den bereits erwähnten fluorierten Halogenkohlenwasserstoffen, Dimethyläther und im speziellen Dichlorfluormethan, Trichlorfluormethan, 1,1,2,2-Dichlortetrafluoräthan und Mischungen derselben eingesetzt werden.

Anstelle der vorgeschlagenen Polyäthylenglykole, insbesondere der Mischungen aus Polyäthylenglykol 400 und 600 lassen sich auch partielle Fettsäureester des Sorbitans bzw. des Polyhydroxiäthylensorbitans, sowie Polyvinylpyrolidone, Polyvinylalkohole, Polyhydroxyäthylenfettalkoholäther bzw.Polyhydroxyäthylenfettsäureester sowie Polyhydroxyäthylenpolyhydroxypropylenkondensate und im besonderen Glycerin-Polyäthylenglykoloxystearat verwenden. Auch äthoxylierte Triglyceride eignen sich als Lösungsmittel für den Wirkstoff. Im besonderen können auch Polyroxyäthylenfettalkoholäther, Polyoxyäthylensorbitanfertsäureester oder Polyoxyäthylenstearinsäureester zu diesen Zwecken herangezogen werden. Weiters können neben Süßstoffen (Natriumsaccharat auch Aromastoffe und gegebenenfalls auch Farbstoffe, wie z.B. Curcumin oder auch ätherische Öle (Pfefferminzöl) eingesetzt werden.

### Beispiel 2:

Ein im Tierversuch als besonders rasch resorbierbar und wirksam gewordener Spray hat folgende konkrete Zusammensetzung: 0,05 g Nifedipin, 1,0 g Glycerin-Polyäthylenglykoloxystearat, 2,0 g Äther, 2,0 g Trichlorfluormethan und 5,0 g Dichlordifluormethan. Dieser Spray hat bereits Sekunden nach der Verabreichung deutliche pharmakologische Wirksamkeit im Tierversuch ergeben. Diese Zusammensetzung zeichnet sich vor allen Dingen dadurch aus, daß sie eine ausgeprägte Dosis Wirkungsbeziehung erkennen ließ und daß tatsächlich bei Verdoppelung der Dosierung ein deutlicher Unterschied im Effekt beobachtet werden konnte. Der Effekt wurde hiebei im besonderen an Hand der Werte für den Bronchialwiderstand verifiziert. In vitro zeigte sich bei dieser Zusammensetzung eine gute Wassermischbarkeit, welche dadurch manifest wurde, daß Sprühstöße, die in Wasser eingebracht wurden, eine klare Lösung hinterließen.

Derartige pharmazeutische Zubereitungen, die sich besonders für die inhalatorische Verabreichung eignen, konnten generell im Bereich von 0,2 bis 5 Gew.-% Nifedipin, 2 bis 20 Gew.-% Glycerin-Polyäthylenglykoloxystearat, 0 bis 25 Gew.-% Diäthyläther und/oder Trichlorfluormethan, Rest Äthanol/Treibmittelgemische aufgefunden werden. In allen Fällen können die genannten Hilfsstoffe zusätzlich eingesetzt werden.

## Patentansprüche

1. Dosierbar versprühbare, pharmazeutische Zubereitung mit einem in einem Lösungsmittel gelösten Wirkstoff, dadurch gekennzeichnet, daß Nifedipin in einem Lösungsmittel, ausgewählt aus der Gruppe Polyäthylenglykole, insbesondere der Mischungen aus Polyäthylenglykol 400 und 600, partielle Fettsäureester des Sorbitans bzw. des Polyhydroxyäthylensorbitans, sowie Polyvinylpyrrolidone, Polyvinylalkohole, Polyhydroxyäthylenfettalkoholäther bzw. Polyhydroxyäthylenfettsäureester sowie Polyhydroxyäthylenpolyhydroxypropylenkondensate und im besonderen Glycerin-Polyäthylenglykoloxystearat gelöst ist, wobei sie 2 bis 5 Gew.-% Nifedipin, 25 bis 40 Gew.-% Lösungsmittel, 40 bis 25 Gew.-% Äthanol und 30 bis 50 Gew.-% Treibmittel enthält, wobei das Treibmittel gegebenenfalls teilweise durch Diäthyläther ersetzt ist.

2. Pharmazeutische Zubereitung nach Anspruch 1, dadurch gekennzeichnet, daß in der Zubereitung etwa 35 Gew.-% Treibmittel enthalten sind.

3. Dosierbar versprühbare, pharmazeutische Zubereitung mit einem in einem Lösungsmittel gelösten Wirkstoff, dadurch gekennzeichnet, daß Nifedipin in einem Lösungsmittel, ausgewählt aus der Gruppe Polyäthylenglykole, insbesondere der Mischungen aus Polyäthylenglykol 400 und 600, partielle Fettsäureester des Sorbitans bzw. des Polyhydroxyäthylensorbitans, sowie Polyvinylpyrrolidone, Polyvinylalkohole, Polyhydroxyäthylenfettalkoholäther bzw. Polyhydroxyäthylenfettsäureester sowie Polyhydroxyäthylenpolyhydroxypropylenkondensate und im besonderen Glycerin-Polyäthylenglykoloxystearat gelöst ist, wobei sie 0,2 - 5 Gew.-% Nifedipin, 2 - 20 Gew.-% Lösungsmittel, 0 - 25 Gew.-% Diäthyläther und/oder Halogenkohlenwasserstoff, Rest Äthanol/Treibmittelgemische enthält.

4. Pharmazeutische Zubereitung nach Anspruch 3, dadurch gekennzeichnet, daß die Wirkstoffdosis für inhalatorische Anwendung je Applikation mit 0,2 - 5 mg, insbesondere 0,5 - 5 mg, für sublinguale Anwendung mit 2-20 mg gewählt wird.

5. Pharmazeutische Zubereitung nach einem der Ansprüche 1 bis 4, dadurch gekennzeichnet, daß die gelösten Wirkstoffe Partikel als Tröpfchen mit 0,2 - 10 µ, vorzugsweise 0,5 - 5 µ, in Emulsion im Trägergasstrom vorliegen.

## Claims

1. A pharmaceutical preparation sprayable in a metered manner and having an active ingredient dissolved in a solvent, characterized in that nifedipine is dissolved in a solvent selected from the group of polyethylene glycols, in particular mixtures of polyethylene glycol 400 and 600, partial fatty acid esters of sorbitan or polyhydroxyethylenesorbitan, and polyvinylpyrrolidones, polyvinylalcohols, polyhydroxyethylene fatty alcohol ethers or polyhydroxyethylene fatty acid esters and polyhydroxyethylene polyhydroxypropylene condensates and in particular glycerin polyethyleneglycol hydroxystearate, and it contains from 2 to 5% by weight of nifedipine, from 25 to 40% by weight of solvent, from 40 to 25% by weight of ethanol and from 30 to 50% by weight of propellant, the propellant being partially replaced where appropriate by diethyl ether.

2. A pharmaceutical preparation according to Claim 1, characterized in that approximately 35% by weight of propellant is contained in the preparation.

3. A pharmaceutical preparation sprayable in a metered manner and having an active ingredient dissolved in a solvent, characterized in that nifedipine is dissolved in a solvent selected from the group of polyethyleneglycols, in particular mixtures of polyethyleneglycol 400 and 600, partial fatty acid esters of sorbitan or polyhydroxyethylenesorbitan, and polyvinylpyrrolidones, polyvinylalcohols, polyhydroxyethylene fatty alcohol ethers or polyhydroxyethylene fatty acid esters and polyhydroxyethylene polyhydroxypropylene condensates and in particular glycerin polyethyleneglycol hydroxystearate, and it contains from 0.2 to 5% by weight of nifedipine, from 2 to 20% by weight of solvent, from 0 to 25% by weight of diethyl ether and/or a halogen hydrocarbon, and the remainder ethanol/propellant mixtures.

4. A pharmaceutical preparation according to Claim 3, characterized in that the dose of active ingredient is selected at 0.2 to 5 mg, in particular 0.5 to 5 mg, per application for administration by inhalation, [and] at 2 to 20 mg for sublingual administration.

5. A pharmaceutical preparation according to any one of Claims 1 to 6, characterized in that the dissolved active ingredients are particles present as droplets with from 0.2 to 10 µ, preferably from 0.5 to 5 µ, in emulsion in the carrier gas stream.

## Revendications

1. Préparation pharmaceutique nébulisable et dosable, contenant une substance active dissoute dans un solvant, caractérisée en ce que la nifedipine est dissoute dans un solvant choisi dans l'ensemble formé par des polyéthylèneglycols, en particulier des mélanges de polyéthylèneglycol 400 et 600, des esters partiels d'acides gras du sorbitanne ou du polyhydroxyéthylènesorbitanne, ainsi que des polyvinylpyrrolidones, des poly(alcools vinyliques), des éthers d'alcools gras polyhydroxyéthyléniques ou des esters d'acides gras polyhydroxyéthyléniques ainsi que des produits de condensation polyhydroxyéthylène-polyhydroxypropylène et en particulier le stéarate de glycérol-polyoxyéthylèneglycol, la préparation contenant 2 à 5 % en poids de nifedipine, 25 à 40 % en poids de solvant, 40 à 25 % en poids d'éthanol et 30 à 50 % en poids de propulseur, le propulseur pouvant éventuellement étre remplacé en partie par de l'éther diéthylique.

2. Préparation pharmaceutique selon la revendication 1, caractérisée en ce que la préparation contient environ 35 % en poids de propulseur.

3. Préparation pharmaceutique nébulisable et dosable, contenant une substance active dissoute dans un solvent, caractérisée en ce que la nifedipine est dissoute dans un solvant choisi dans l'ensemble formé par des polyéthylèneglycols, en particulier des mélanges de polyéthylèneglycol 400 et 600, des esters partiels d'acides gras du sorbitanne ou du polyhydroxyéthylènesorbitanne, ainsi que des polyvinylpyrrolidones, des poly(alcools vinyliques), des éthers d'alcools gras polyhydroxyéthyléniques ou des esters d'acides gras polyhydroxyéthyléniques ainsi que des produits de condensation polyhydroxyéthylène-polyhydroxypropylène et en particulier le stéarate de glycérol-polyoxyéthylèneglycol, la préparation contenant 0,2 à 5 % de nifedipine, 2 à 20 % en poids de solvant, 0 à 25 % en poids d'éther diéthylique et/ou d'un hydrocarbure halogéné, le reste étant de l'éthanol/des mélanges de propulseur.

4. Préparation pharmaceutique selon la revendication 3, caractérisée en ce que la dose de substance active, par application, est choisie à 0,2 - 5 mg pour une administration par inhalation, en particulier 0,5 à 5 mg, et à 2-20 mg pour une administration sublinguale.

5. Préparation pharmaceutique selon l'une des revendications 1 à 4, caractérisée en ce que les particules des substances actives dissoutes sont présentes sous forme de gouttelettes de 0,2 à 10 µ, avantageusement 0,5 à 5 µ, en émulsion dans le courant de gaz porteur.
